# EUROPEAN PATENT SPECIFICATION

(11) **EP 2 694 969 B1**
(45) Date of publication and mention of the grant of the patent: **01.07.2015**
(21) Application number: 12712128.3
(22) Date of filing: 05.04.2012
(51) Int. Cl.: G01N 33/68, G01N 33/564

(54) **ANTI-VE-CADHERIN AUTOANTIBODIES AS A BIOMARKER OF VASCULAR ALTERATIONS ASSOCIATED WITH DISORDERS**
ANTI-VE-CADHERIN-ANTIKÖRPER ALS BIOMARKER VASKULÄRER VERÄNDERUNGEN IM ZUSAMMENHANG MIT ERKRANKUNGEN
AUTO-ANTICORPS ANTI-VE-CADHÉRINE EN TANT QUE MARQUEUR BIOLOGIQUE D'ALTÉRATIONS VASCULAIRES ASSOCIÉES À DES TROUBLES

(30) Priority: 08.04.2011 EP 11305418
(43) Date of publication of application: 12.02.2014
(73) Proprietor: Institut National de la Santé et de la Recherche Médicale (INSERM), 75013 Paris (FR); Centre Hospitalier Universitaire de Grenoble, 38043 Grenoble Cedex 9 (FR); Université Joseph Fourier, 38041 Grenoble Cedex 9 (FR)
(72) Inventor: VILGRAIN, Isabelle, F-38054 Grenoble Cedex 09 (FR); GULINO-DEBRAC, Danielle, F-38054 Grenoble Cedex 09 (FR); BOUILLET, Laurence, F-38054 Grenoble Cedex 09 (FR)
(74) Representative: Hirsch, Denise Marie
(86) International application number: PCT/EP2012/056371
(87) International publication number: WO 2012/136820

(56) References cited:
- DUVAL ARNAUD ET AL: "Endothelial dysfunction in systemic lupus patients with low disease activity: evaluation by quantification and characterization of circulating endothelial microparticles, role of anti-endothelial cell antibodies", RHEUMATOLOGY, OXFORD UNIVERSITY PRESS, LONDON, GB, vol. 49, no. 6, 1 June 2010 (2010-06-01), pages 1049-1055, XP008137138, ISSN: 1462-0324
- anonymous: "Systemic lupus erythematosus", , 3 November 2010 (2010-11-03), XP007918789, Retrieved from the Internet: URL:http://web.archive.org/web/20101103230 621/http://en.wikipedia.org/wiki/Systemic_ lupus_erythematosus [retrieved on 2011-05-27]

## Description

### FIELD OF THE INVENTION:

The present invention relates to a method for predicting and/or diagnosing vascular alterations associated with a disorder in a patient.

### BACKGROUND OF THE INVENTION:

Autoimmune and inflammatory disorders are characterized by idiopathic systemic vasculitis with the common characteristic of acute or chronic inflammatory compromise of the small and large vessels walls, associated with fibrinoid necrosis (1, 2). The vessel wall is an active, integrated organ composed of endothelial, smooth-muscle, and fibroblast cells coupled to each other in a complex autocrine-paracrine set of interactions. The vasculature is capable of sensing changes within its milieu, integrating these signals by intercellular communication, and changing itself through the local production of mediators that influence structure as well as function (3). Vascular remodeling is an active process of structural alteration that involves changes in at least four cellular processes -- cell growth, cell death, cell migration, and production or degradation of extracellular matrix -- and is dependent on a dynamic interaction between locally generated growth factors, vasoactive and hemodynamic stimuli (4). Although all the vascular cells may participate in the remodelling process, the endothelium is particularly suited to play a prominent part.

Vascular endothelium integrity is tightly controlled by a series of complex interactions between adjacent endothelial cells and by additional interactions between the endothelial cells (5). Disruption of these interactions results in leakage of plasma constituents into the surrounding tissues and oedema. Vascular endothelial cells express a unique member of the cadherin family, termed VE-cadherin (cadherin-5), which has been shown to play an important role in the establishment and maintenance of endothelial monolayer integrity (6, 7). Cadherins consist of 5 extracellular domains (EC1-5) and are anchored to the actin cytoskeleton through their cytoplasmic tail. The vascular endothelial-specific (VE)-cadherin mediates homophilic adhesion between neighbouring endothelial cells and is localized within specialized structures at cell-cell contacts, called adherens junctions, and is constitutively expressed throughout the entire vasculature (8). Accumulating evidence implicates VE-cadherin in various aspects of vascular biology including endothelial cell migration (9), survival (10) contact-induced growth inhibition (11), vascular integrity (12) and, most notably, endothelial-cell assembly into tubular structures (13). The importance of VE-cadherin in developmental angiogenesis has been demonstrated by the severe impairment of vascular assembly in VE-cadherin null embryos, leading to embryonic lethality by day E9.5 (14, 15). Upon endothelial cell stimulations, VE-cadherin post-translational modifications have been reported. Indeed, tyrosine phosphorylation in the cytoplasmic domain or cleavage of the extracellular domain of protein are correlated with increased permeability (16-20).

Of importance, antibodies developed against various VE-cadherin extracellular regions have also been found for their ability to bind to specifically exposed VE-cadherin epitopes in tumor angiogenesis. Thus targeting tumoral vasculature was described to inhibit tumor growth as well as blockade of adherens junction formation in vitro, and disruption of established vessels, and inhibition of vascular assembly *in vivo* (21-25).

In dysimmune diseases, several autoantibodies have been characterized such as antineutrophil cytoplasmic autoantibodies (ANCA) in Wegener's granulomatosis nuclear antigen (ANA) as well as to centromere and scl70/topoisomerase in systemic lupus erythematosus (SLE) or sclerodermia, anti citrunillated protein and anti phospholipid antibodies in rhumathoid arthrithis (26). These autoantibodies are widely accepted as pathogenic and are believed to promote several syndroms such as thrombosis. On the other hand, several studies have characterized the presence of autoantibodies to endothelial cells and their link with the severity of several dysimmune diseases such as lupus erythematosus and scleroderma (27, 28). In addition, SLE was the first disease in which antibodies to endothelial cells (AECA) were discovered in 1971 and were suspected to be involved in vascular attacks (29).

Many investigators have suggested that the adhesive interactions of leucocytes with endothelial cells and with the extracellular matrix have a central role in the function of the immune system. Thus, VE-cadherin modifications in inflammatory and angiogenic processes might be involved in the appearance of autoantibodies to specific regions of the protein. This hypothesis has never been explored in human diseases while several antibodies against VE-cadherin have been produced for research and use in cell culture and in mice models to target tumoral endothelium (21-25).

However, up to now, the identification of human VE-cadherin autoantibodies has never been reported in patients affected with a dysimmune disease or cancer disorders.

DUVAL ARNAUD ET AL: "Endothelial dysfunction in systemic lupus patients with low disease activity: evaluation by quantification and characterization of circulating endothelial microparticles, role of anti-endothelial cell antibodies", RHEUMATOLOGY, OXFORD UNIVERSITY PRESS, LONDON, GB, vol. 49, no. 6, 1 June 2010 (2010-06-01), pages 1049-1055 disclose anti-endothelial cell antibodies (AECA) and endothelial microparticles (EMps) as markers in systemic lupus (SL). According to D1 EMps with CD144 were hardly detectable (see p. 1051, col. 2, para. 3; CD144 is a synonym for VE-cadherin) and AECAs in general appear to play only a minor role (see abstract, last para.).

For dysimmune diseases including connective tissue diseases (e.g. lupus and sclerodermia), other specific auto antibodies are currently used for the diagnosis but not for detecting vascular endothelium alterations associated with. Furthermore, vascular endothelium exploration is difficult. Some antibodies may be associated with some vascular alterations (e.g. anti endothelial cells antibodies, antiphospholipids antibodies, anti endostatine...) but they do not have good specificity and sensibility.

Furthermore, due to the seriousness of dysimmune diseases as well as cancer disorders and the risk to develop severe vascular complications, but also to the high costs of treating it, an early diagnosis is, obviously, extremely desirable: it would contribute to preventing the rapid progression of the disorder to severe stage with vascular complications. Identifying the individuals at risk of developing such vascular complications is therefore a necessity. Knowledge of one disorder severity predictor would be indeed highly desirable to generate predictive models that can aid clinicians 'decision making, in particular by identifying patients who present severe vascular complications or who are at high risk of developing such complications following dysimmune disorders or cancer disorders.

Therefore, there is still a need for biomarkers useful for diagnosis and predicting the vascular alterations associated with these diseases.

### SUMMARY OF THE INVENTION:

The present invention relates to a method for predicting and/or diagnosing vascular alterations associated with a disorder in a patient, comprising a step of detecting or quantifying the presence of anti-VE-cadherin autoantibodies in a biological sample obtained from said patient.

The present invention also relates to a kit suitable for carrying out the method of the invention, comprising a solid support coated with at least one VE-cadherin antigen and at least a labelled antibody specifically recognizing antibodies.

### DETAILED DESCRIPTION OF THE INVENTION:

The inventors have now identified that anti-VE-cadherin autoantibodies is a biomarker for predicting and/or diagnosing, in particular in serum, vascular alterations associated with diverse disorders or diseases. Indeed, they have detected and quantified anti-VE-cadherin autoantibodies in sera of patients affected with various diseases and disorders. They have also characterized these autoantibodies by demonstrating after their purification they may be pathogenic and contribute to vascular injury in several diseases.

### Definitions:

Throughout the specification, several terms are employed and are defined in the following paragraphs.

As used herein, the term "VE-cadherin" has its general meaning in the art and corresponds to vascular endothelial cadherin. This protein of 784 amino acids is an endothelial-specific cadherin localized at the intercellular junctions of most organs and tissues. By way of example, human VE-cadherin is provided under GenBank accession number CAA56306 and has been described in the international application WO 98/25946. VE-cadherin comprises an extracellular domain, which consists of five cadherin-like repeats, a transmembrane domain and a short cytoplasmic tail as previously described (24).

As used herein, the term "antibody" refers to a polypeptide (or protein) capable of specifically binding an antigen, typically and preferably by binding an epitope or antigenic determinant present on said antigen.

As used herein, the terms "antigen" or "VE-cadherin antigen" refer to VE-cadherin (glycosylated or not glycosylated) or a fragment which is specifically recognized and bound by anti-VE-cadherin autoantibodies (also called VE cadherin Ig). It must be further noted that VE-cadherin antigen, such as VE-cadherin extracellular domain, may also be generated *in vitro* by genetic manipulation and produced in heterologous systems (such as bacteria).

The term "fragment" of a reference sequence refers to a chain of contiguous nucleotides or amino acids that is shorter than the reference sequence. Thus, this term includes peptide of several amino acids as well as the whole VE-cadherin extracellular domain. As used herein, the term "peptide" has the meaning usually given in the art. More specifically, the dividing line between proteins and peptides is usually set at a length of approximately 50 amino acids. Thus the peptides according to the invention preferably have a length of at most 50, 40, 35, 30, 25, 20, 15 or 10 amino acids.

As used herein, "detecting" means determining if anti-VE-cadherin autoantibodies are present or not in a biological sample and "quantifying" means determining the amount of anti-VE-cadherin autoantibodies in a biological sample.

As used herein, the term "diagnosing" refers to methods by which the person skilled in the art can estimate and even determine whether or not a subject is suffering from a given disorder or condition. The person skilled in the art often makes a diagnosis on the basis of one or more diagnostic indicators, such as anti-VE-cadherin autoantibodies, the level (including presence or absence) of which is indicative of the presence or absence of vascular alterations related with a disorder such as dysimmune diseases or cancer disorders.

As used herein, the terms "vascular alterations", "vascular complications", "vascular perturbations" or "vascular injury" refers to an abnormal and/or undesirable change to vascular integrity. Perturbations of vascular integrity can be manifested in the form of one or more tissue and cellular conditions, including but not limited to those associated with endothelial cell necrosis, endothelial cell apoptosis, trauma to the endothelium, injury, vascular leakage, hypertension, and vascular damage. For example, inflammation of the vasculature is a common cause associated with alterations of vascular integrity. The vascular alterations include for instance the vasculitis (e.g. Behçet disease, Wegener disease, Horton disease, rheumatoid purpura and cryoglobulinemia). A further example include trauma affecting the vascular endothelium, e.g. trauma (such as injuries) to the blood vessels, including the vascular network of organs, to which a mammal or a human is subjected. Trauma includes conditions caused by internal events as well as those that are imposed by an extrinsic agent such as a pathogen. For example, trauma affecting the vascular endothelium includes the disorganization of endothelial cell-to-cell adherens junctions and therefore of the endothelial cell-to-cell contacts leading subsequently to vascular permeability caused by autoantibodies recognizing VE-cadherin, a major components of adherens junctions.

As used herein, the terms "vascular" or "vasculature" refer to the system of vessels carrying blood throughout the mammalian body.

A "disorder" is any condition that would benefit from a method of the invention and therefore in which a vascular alteration may happen. This includes chronic and acute disorders or diseases. Non-limiting examples of disorders or diseases include but are not limited to, angiogenic disorder (e.g., cancer disorder), and dysimmune disease (e.g., inflammatory disorder and autoimmune disease). It should be noted that disorders include also pathological conditions associated with dysregulation of angiogenesis.

The term "angiogenesis", as used herein, refers to a cellular event resulting in neovascularization, in which vascular endothelial cells proliferate, prune and reorganize to form new vessels from preexisting vascular networks.

As used herein, an dysimmune (autoimmune or autoinflammatory) refers to conditions triggered by aberrant reactions of the human immune and inflammatory system

A "patient" in the context of the present invention is a human (male or female). Typically said patient has been previously diagnosed with a disease or disorder (e.g. a dysimmune disease (such as autoimmune or inflammatory disease) or a cancer disease).

As used herein, a "biological sample" refers to blood, serum or plasma, and urine, saliva, cerebrospinal fluid, ascites, pleural effusion, obtained from a patient to be tested.

The term "biomarker", as used herein, refers generally to a molecule, i.e., a gene (or nucleic acid encoding said gene), protein, the expression of which in a biological sample from a patient can be detected by standard methods in the art (as well as those disclosed herein), and is predictive or denotes a condition of the patient from which it was obtained.

### Diagnostic methods and kits:

A first aspect of the present invention relates to a method for predicting and/or diagnosing vascular alterations associated with a disorder in a patient, comprising a step of detecting or quantifying the presence of anti-VE-cadherin autoantibodies in a biological sample obtained from said patient.

In one embodiment, the disorder is selected in the group consisting of cancer disorder and dysimmune disease (e.g., inflammatory disorder and autoimmune disease).

In a particular embodiment, the dysimmune disease is an inflammatory disorder.
Accordingly, the inflammatory disorder is selected in the group consisting of sarcoidosis and still disease.

In a particular embodiment, the dysimmune disease is an autoimmune disease.
In a preferred embodiment, said autoimmune disease is a connective tissue disease.
Accordingly, the connective tissue disease is selected in the group consisting of systemic lupus erythematosus (SLE), rheumatoid arthritis (RA), Sjögren's syndrome, scleroderma, dermatomyositis or mixed connective-tissue disease (MCTD).

In another particular embodiment, the cancer disorder is a solid cancer.
Accordingly, the solid cancer is preferably a highly vascularised cancer (e.g., breast cancer and kidney cancer including metastatic kidney cancer).

In one embodiment, the biological sample is blood, serum or plasma sample.
In a preferred embodiment, the biological sample is serum sample

The amount of anti-VE-cadherin autoantibodies may indeed be indicative of the risk for said patient of having vascular alterations associated with a disorder and/or of the presence of vascular alterations and therefore allow to evaluate the severity of said disorders. Thus, a tested patient may for instance be classified as (i) a patient having a stable disorder or with a slow progression, or (ii) a patient having a high risk of developing vascular complications.

In another embodiment, said method for predicting and/or diagnosing vascular alterations associated with a disorder in a patient, comprises the steps of:
(a) contacting at least one VE-cadherin antigen with a biological sample obtained from said patient;
(b) quantifying the level of anti-VE-cadherin autoantibodies in said biological sample; and
(c) comparing the amount of anti-VE-cadherin autoantibodies with a reference level; wherein an increased amount with regard to the amount in the control sample is indicative of a risk of developing vascular alterations or the presence of vascular alterations.

The amount of anti-VE-cadherin autoantibodies quantified may thus be compared with the corresponding amount detected in the biological samples of control subjects, in biological previous samples obtained from the subject or with normal reference values.
While the method of the invention is intended for predicting and/or diagnosing vascular complications associated with a disorder, control subjects are for example subjects that have not been diagnosed for one of said disorders. Normal reference values refer to the amount of anti-VE-cadherin autoantibodies that can be determined by the method of the invention in a subject that has not been diagnosed for said disorder.
In one embodiment, said control value or reference value is determined by using the average values obtained from at least 10, preferably from at least 100 control subjects.

Further, in some embodiments, multiple determinations of the anti-VE-cadherin autoantibodies over time can be made to facilitate diagnosing as well as monitoring. A temporal change in the anti-VE-cadherin autoantibody level can be used to predict a clinical outcome, monitor the progression of the vascular alterations associated with a disorder and/or efficacy of appropriate therapies directed against said vascular alterations and/or disorder.
In such an embodiment for example, one might expect to see a decrease in the level of anti-VE-cadherin autoantibodies (and potentially at least one additional biomarker) in a biological sample over time during the course of effective therapy.

In another embodiment of the invention, the method for diagnosing and/or monitoring according to the invention also comprises the detection and/or the quantification of at least one additional marker (e.g. other autoantibody) useful to predicting or diagnosing vascular alterations associated with a disorder.

### General methods for detecting or quantifying the presence of anti-VE-cadherin autoantibodies in a biological sample

According to the invention, the detection or quantification of anti-VE-cadherin autoantibodies in a biological sample is achieved by any methods known in the art.

Examples of said methods include, but are not limited to, standard electrophoretic and immunodiagnostic techniques such as western blots, immuno-precipitation assay, radioimmunoassay, ELISA (enzyme-linked immunosorbant assay), "sandwich" immunoassay, immunoradiometric assay, gel diffusion precipitation reaction, immunodiffusion assay, precipitation reaction, agglutination assay (such as gel agglutination assay, hemagglutination assay, etc.), complement fixation assay, protein A assay, immunoelectrophoresis assay, high performance liquid chromatography, size exclusion chromatography, solid-phase affinity, etc

In an embodiment, the detection or quantification of the presence of anti-VE-cadherin autoantibodies is carried out by using at least one VE-cadherin antigen.
Examples of said VE-cadherin antigen include, but are not limited to, the different fragments of the VE-cadherin extracellular domain CAD 1, CAD 2, CAD 3, CAD 4, CAD 1-2, CAD 1-3, CAD 1-4, CAD 2-3, CAD 2-4 and CAD 3-4.
In a particular embodiment, the VE-cadherin antigen is the VE-cadherin CAD 1-4 fragment delimited by the amino acids situated at positions 1 and 431 of the VE-cadherin extracellular domain.
In another particular embodiment, the VE-cadherin antigen is VE-cadherin extracellular domain (CAD1-CAD5).

In another embodiment, VE-cadherin antigen(s) may be immobilized onto a solid support, such as for example protein coupling or protein binding surface (e.g., column matrix or well of a microtiter plate), a membrane (e.g., nitrocellulose, PVDF or similar material), colloid metal particles, iron oxide particles, or polymeric beads. One example of polymeric beads is a latex particle. For example, the detection or quantification of anti-VE-cadherin autoantibodies in a sample may thus be achieved by a cytometric bead array system wherein the antigen specifically bound by anti-VE-cadherin autoantibodies is coated directly or indirectly on beads.

In such embodiment, the antigen is bound to or coated on a solid phase support using standard non-covalent or covalent binding methods, depending on the required analytical and/or solid phase separation requirements. The solid support may be in the form of test-tubes, beads, microparticles, filter paper, membrane, glass filters, magnetic particles, glass or silicon chips or other materials known in the art. The use of microparticles, particularly magnetisable particles, that have been directly coated with the antigen or particles that have been labelled with a universal binder (such as avidin) is useful for significantly shortening the assay incubation time.

According to the invention, a detecting antibody that specifically binds to antibodies may be used. Said detecting antibody thus binds to anti-VE-cadherin autoantibodies via for example Fc portions. Detecting antibodies useful in the various embodiments of the invention encompass commercially available antibodies and antibody fragments, as well as any novel antibodies generated to bind to other antibodies such as anti-VE-cadherin autoantibodies. The antibodies used in various embodiments exemplified herein are monoclonal or polyclonal in nature. Other antibodies and antibody fragments, such as recombinant antibodies, chimeric antibodies, humanised antibodies, Fab or Fv fragments are also useful.
In a particular embodiment, the detecting antibody is an anti-human antibody such as e.g. a human IgG antibody.

In one embodiment, said detecting antibody may be labelled with a detectable molecule or substance. Examples of suitable labels for this purpose include a chemi luminescent agent, a colorimetric agent, an energy transfer agent, an enzyme, a substrate of an enzymatic reaction, a fluorescent agent, or a radioisotope. Examples of chemiluminescent agent include an enzyme that produces a chemiluminescent signal in the presence of a substrate(s) that produce chemiluminescent energy when reacted with the enzyme. Examples of such an enzyme include horseradish peroxidase (HRP) and alkaline phosphatase (AP). Other examples of a chemiluminescent agent include a non-enzymatic direct chemiluminescent label, such as Acrinidium ester system. Examples of a colorimetric agent include an enzyme such as horseradish peroxidase, alkaline phosphatase, and acetylcholine esterase (AChE). Examples of energy transfer agent include fluorescent lanthanide chelates. Examples of fluorescent agents include fluorescent dyes. Examples of radioisotopes include ¹²⁵I, ³⁵S, ³²P, ¹⁴C and ³H. The label may be coupled directly or indirectly by any known method in the art.

In an embodiment, the detection or quantification of anti-VE-cadherin autoantibodies in a biological sample may be achieved by a protein chip array system, wherein VE-cadherin antigen is coated directly or indirectly on a protein chip array. The sample to be tested is labelled by biotinylation in vitro. Biotinylated anti-VE-cadherin autoantibodies trapped on the array are then detected by avidin or streptavidin which strongly binds biotin. If avidin is conjugated with horseradish peroxidase or alkaline phosphatase, the captured anti-VE-cadherin autoantibodies can be visualized by enhanced chemical luminescence. The amount of anti-VE-cadherin autoantibodies bound to the VE-cadherin antigen represents the amount of anti-VE-cadherin autoantibodies in the sample. Other methods, like immunochemical staining, surface plasmon resonance, matrix-assisted laser desorption/ionization-time of flight, can also be used to detect the captured anti-VE-cadherin autoantibodies.

In another embodiment of the invention, the quantification of anti-VE-cadherin autoantibodies in a biological sample may be achieved by homogeneous time resolved fluorescence (HTRF).
In one embodiment, a VE-cadherin antigen is coupled with a donor fluorophore, such as Europium cryptate (Eu3+ cryptate) or Lumi4™-Tb (Tb2+ cryptate), and a detecting antibody directed to antibodies (binding for example Fc portions) is coupled with an acceptor such as XL665, a modified allophycocyanin, or D2 which represents a second generation of acceptor characterized by an organic structure 100 times smaller.
In an alternative embodiment, the VE-cadherin antigen is coupled with an acceptor and the detecting antibody is coupled with a donor fluorophore.
When these two fluorophores are brought together by a biomolecular interaction, a portion of the energy captured by the donor fluorophore during excitation is released through fluorescence emission at 620nm, while the remaining energy is transferred to the acceptor. This energy is then released by the acceptor as specific fluorescence at 665 nm.

### Kits according to the invention

The invention is further directed to a kit suitable for carrying out the methods according to the invention. Such a kit may comprise:
- a solid support coated with at least one VE-cadherin antigen (such as e.g the VE-cadherin extracellular domain or a fragment thereof as defined above); and
- at least a labelled antibody specifically recognizing antibodies (such as e.g an anti-human IgG antibody).

The kit may further comprise one or more biochemical reagents useful for carrying out the method according to the invention such as e.g. a buffer solution such as PBS and a wash buffer.

The invention will be further illustrated by the following figures and examples. However, these examples and figures should not be interpreted in any way as limiting the scope of the present invention.

### FIGURES:

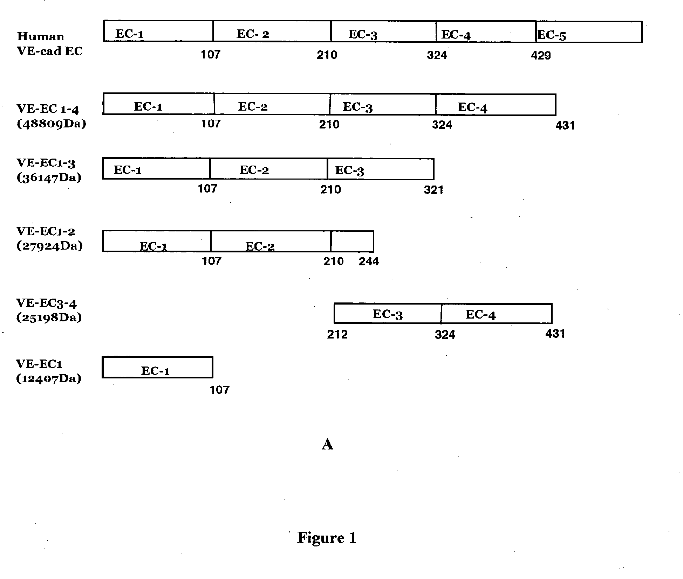

**Figure 1****: Schematic representation of recombinant VE-cadherin fragments.**

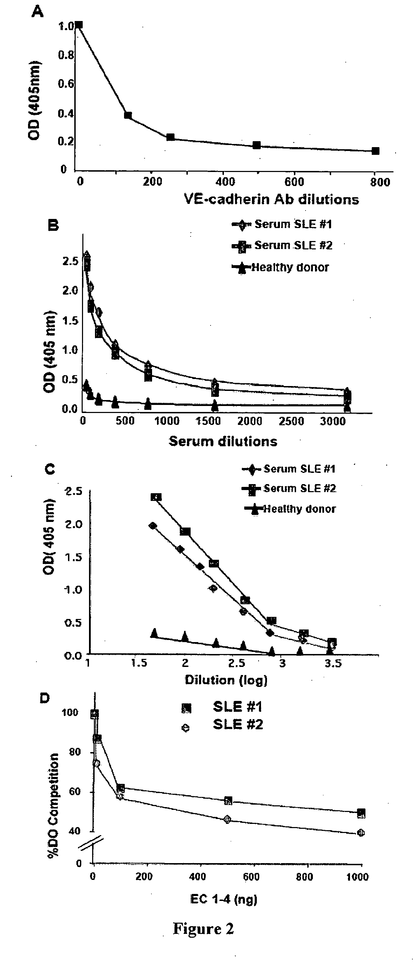

**Figure 2****: ELISA for Anti VE-cadherin antibodies detection:** The recombinant human VE-cadherin fragment EC 1-4 was used as capture antigen for the coating of 96 well plates. **A**. The anti VE-cadherin antibody (BV9) was used to test the ELISA using serial dilutions of the antibody. All the assays were run in duplicates. **B:** Serum Dilution. Detection of anti-VE cadherin in sera from two lupic patients, by ELISA as described in "materials and methods". Different serum dilutions from 1:50, to 1:1600 were analyzed by ELISA. For each patient, the average of two OD is represented as a single point for each dilution. Two SLE patient and a healthy donor sera were tested using increased serum serial dilution. **C:** Logarithmic representation of the data in B showed the quantitative and linear regression curve for quantification. **D:** Neutralization assay using increasing concentrations of EC1-4 added to human serum samples before ELISA. All the assays were run in duplicates.

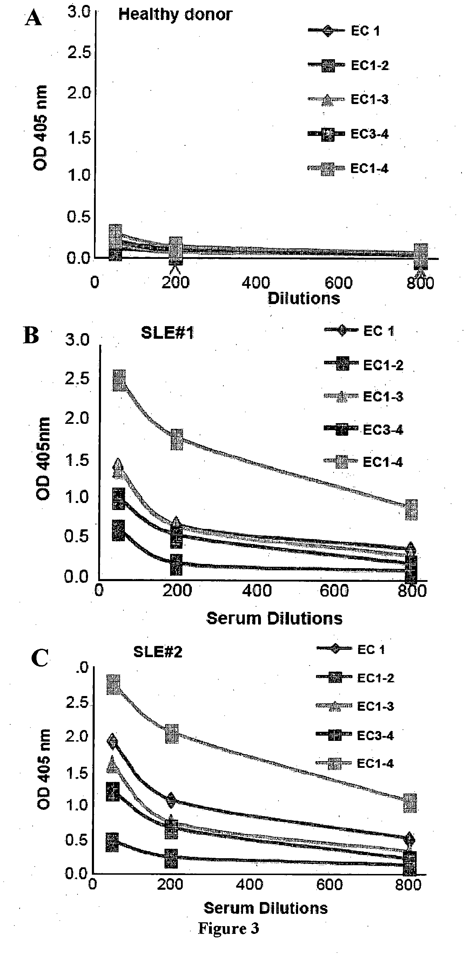

**Figure 3****: Epitope mapping of purified IgG from SLE and healthy donor determined by ELISA and Dot Blot analysis:** Characterization of the epitopes by ELISA : The VE-cadherin chimera fragments EC 1-GST, EC 1-2, EC1-3, EC 3-4, EC 1-4 were plated at equimolar concentration (0.2µM) and the ELISA was performed using three dilutions (1/50, 1/200 and 1/800) of patients IgG from healthy donor (**A**) and two SLE patients (**B, C**).

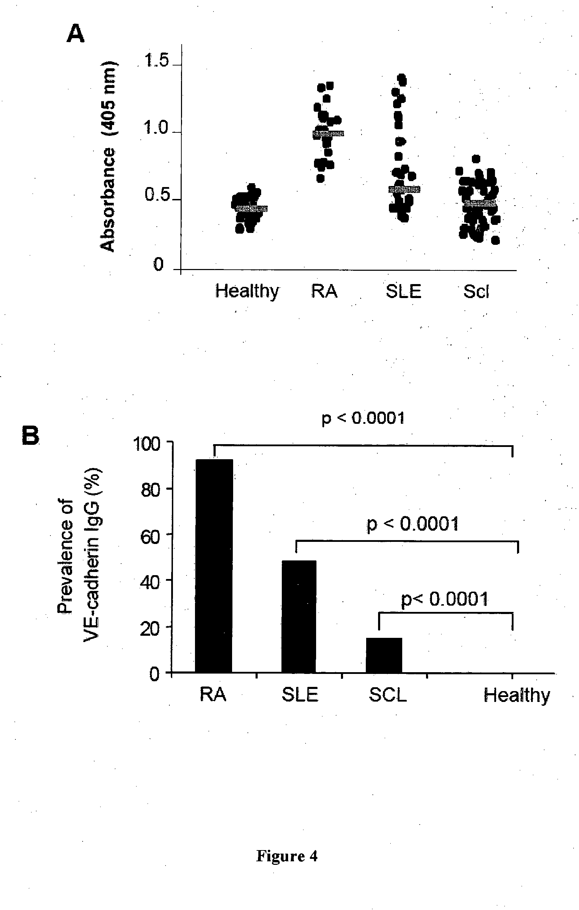

**Figure 4****: Prevalence of anti-VE cadherin IgG in patients with autoimmune** diseases. **A:** Sera from healthy controls (healthy), patients with rheumatoid arthritis (RA), patients with lupus (SLE), and patients with sclerodermia (scl) were analyzed for anti-VE cadherin antibodies by ELISA. Each symbol corresponds to the serum of a single subject. All the samples were tested in duplicates. For each patient, the average of two OD is represented as a single point. Bars indicate mean values for each group. **B:** Prevalence of anti-VE cadherin (3SDs) in patients groups. The percentage of patients with anti-VE cadherin IgG titer that exceeds the mean control value by more than 3SDs is shown. The titers of RA (n=63), SLE (n=35), SCL (n=48) patients is significantly (*P* < 0.001 Mann-Whitney test) higher than those of healthy control subjects (*n*=50).

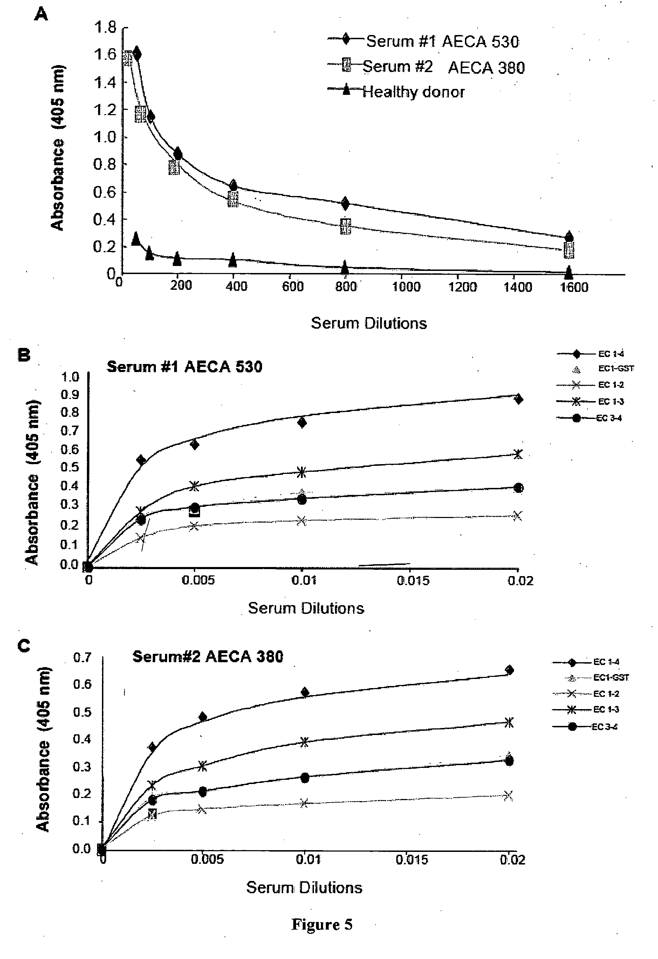

**Figure 5****: Detection of VE-cadherin autoantibodies in patient serum positive for anti endothelial cell antibodies. A :** The serum from two patients positive for AECA (#1=530, #2=380 as measured in Grenoble University Hospital in 2001) were tested for VE-cadherin autoantibodies using ELISA with EC1-4 VE-cadherin fragment. An healthy donor was used as negative control. **B, C:** The two serum were tested for VE-cadherin autoantibodies by ELISA using the five recombinant VE-cadherin fragments.

### EXAMPLE 1: DETECTION OF ANTI-VE-CADHERIN AUTOANTIBODIES IN SERA OF PATIENTS AFFECTED WITH DYSIMMUNES DISEASES

### Material & Methods

***Sources of human sera:*** This study includes 152 adults, consisted of four groups of subjects illustrated in Table 1. All subjects were 18 years of age or older.

50 sera from healthy donors were obtained from the French National Blood Service. 35 sera from patients who fulfilled the American college of rheumatology criteria for SLE (1997) and who had anti nuclear antibodies (100%) were from the Immunology Department, Grenoble University Hospital.

48 sera from patients who fulfilled the American college of rheumatology criteria (1980) for sclerodermia and who had antinuclear antibodies (92%) were obtained from the Vascular Medicine Department, Grenoble University Hospital.

63 sera from rheumatoid Arthritis patients were obtained from the VErA cohort recruited prospectively in the Rheumatology Department of the Rouen University Hospital. Serum samples were collected, aliquoted and stored at 80°C.

All serum samples were kept frozen at 80°C, and then thawed shortly before use.

**Table 1: Characterization of patient population**

| Disease | N#. | Median age (range) | Female/male |
|---|---|---|---|
| Healthy | 50 | 50.5 (35-75) | 25/25 |
| Lupus | 36 | 36 (27-61) | 32/4 |
| Rheumatoid arthritis | 63 | 53 (26-76) | 41/22 |
| Scleroderma | 48 | 51.1 (2-75) | 43/5 |

***Materials:*** ELISA plate microloan (96 wells, Nunc Maxisorp)were from DUTSCHER, France, Goat anti-human IgG-biotin conjugate was from Southern Biotech, Steptavidin-alkaline phosphatase substrate system and paranitrophenyl phosphate liquid were from Sigma.

***Reagents:*** The micro-bicinchonic acid protein assay reagent kit was from Pierce (Oud Beijerland, The Netherlands). Nitrocellulose was obtained from Schleicher and Schuell (Ecquevilly, France). The enhanced chemiluminescence detection reagents were purchased from PerkinElmer (Lifesciences, Belgium). Protein G-Sepharose was from Pharmacia (Netherlands).

***Antibodies:*** Mouse mAbs against the extracellular domain of human VE-cadherin was from [BV9] (ab7047) | Abcam. The polyclonal anti-VE-cadherin antibody Cad 3, was raised against a recombinant VE-cadherin fragment encompassing amino acids 1-258 of VE-cadherin. This antibody was produced and immunopurified as previously described (21).

***Production and expression of VE-cadherin chimeras:*** The bacterially expressed VE-cadherin recombinant fragments VE-EC1-4, EC 1, EC 1-2, EC 1-3 fragments were produced and purified as previously described (21).

***Western Blotting:*** The purified recombinant fragments (2 µg) were analyzed by SDS-PAGE (12% Acrylamide) and then transferred to nitrocellulose membrane using an electro blotting apparatus (BioRad Labs, Richmond, CA, USA) at 110 V for 1 h. The membranes were blocked for 1 h at room temperature with 5% non-fat dry milk in phosphate buffered saline containing 0.05% (v/v) Tween 20. The blots were incubated overnight at 4°C with polyclonal antibodies (rabbit anti VE-cadherin EC 1 fragment. After being washed, the blots were incubated for 1h with goat anti-rabbit HRP or horse radish peroxidase-1 conjugated rabbit anti-human IgG (Sigma, Saint Quentin Fallavier, France) diluted 1:10 000 in PBS-Tween, and incubated at room temperature for 1h. Detection of immunoreactive proteins was done using enhanced chemiluminescence (NEN Life Science Products).

***Dot blot analysis:*** 2 µg of individual VE-cadherin recombinant were spotted onto nitrocellulose membrane and allowed to adhere for at least 2 h at 37°C before blocking for 1 h at room temperature in 5 % (w/v) non fat milk-PBS. Patient sera testing positive in ELISA or control sera diluted five fold were added to the strip and incubated at 4°C overnight. Dotted membrane with no primary antibody served as 'negative control'. Following day the membranes were washed with 0.1% (v/v) Tween-20 in PBS and then incubated with either BV9 monoclonal antibody (0.5 µg/mL) or purified IgG from SLE patient or healthy donor (250 µg/mL) overnight at 4°C. The membranes were then washed three times and incubated in suitably diluted (1: 20,000) goat anti human HRP labeled secondary antibody (SIGMA, France) in 5 % (w/v) non fat milk-PBS for 1 h at room temperature. Membranes were then washed as described above. Detection was done using enhanced chemiluminescence plus (ECL) kit (Amersham, France) as for the standard protocol.

***Affinity Chromatography for Immunoglobulins purification:*** Serum from either healthy donors or SLE patient (4 mL) was diluted with 36 mL phosphate buffer NaH₂PO₄, Na₂HPO₄ 0.2M, pH 7.1 and was incubated in the presence of Protein G Sepharose (2mL) (previously equilibrated with 12mL phosphate buffer NaH₂PO₄, Na₂HPO₄ 0.2M, pH 7.1) in a Falcone tube over night at 4°C under gentle rotation. At the end of the incubation, the gel was poored into a Biorad column (1 x 10 cm), and the flow through was kept. After a column wash with 60 mL of the same buffer, the elution was carried out with with 10 mL glycine buffer 100mM, pH 2.7, and 1mL fractions were collected in Eppendorf tubes containing 100 uL Tris 1M, pH 8. Optical densities at 280 nM were measured on an aliquot of each collected fraction (1 mL) to determine IgG concentrations using DO = ε.C.l (Immunoglobulins Molar Extinction Coefficient, εmolar = 1.45 L/g/cm). The final fractions were dialyzed against PBS overnight at 4°C before use. The same protocol was performed to purify IgG from a healthy donor.

***Cell culture and treatment:*** Human umbilical vein endothelial cells (HUVECs) were isolated as previously described (30). Only first to third passage HUVECs were used in experiments. Cells were maintained in supplemented Dulbecco's modified Eagle's medium (Invitrogen) containing 10% fetal bovine serum, 2 mM L-glutamine, 100 units/ml penicillin, 100 units/ml streptomycin, and 20 ng/mL gentamicin, in a humidified 5% CO₂ incubator at 37 °C. Glass coverslips (13-ram diameter) were coated overnight at 4°C with 7 µg/mL fibronectin then rinsed with serum-free medium 199 before seeding 3.5 x 10⁴ cells/coverslip in 0.5 ml culture medium. HUVECs were grown to confluency for 72 h. The cell monolayers were incubated overnight at 37°C temperature, with 2.5 mg/mL of purified IgG from either healthy donor or SLE patient. The next day the cells were observed with a Zeiss Axioskop 2 microscope.

### Enzyme-linked immunosorbent assay (ELISA) binding for VE-cadherin antibodies:

Ninety six well microtitre plates (Immunoplate MAXISORPN NUNC, DUTSCHER, France) were coated overnight at 4°C with 1 ug /well of purified recombinant VE-cad(EC1-4 or as stated) fragment in carbonate- bicarbonate coating buffer (Na₂CO₃/NaHCO₃ 0,2M pH 9.6). Excess solution was flicked off the next day and modules were washed with PBS containing 0.05% (v/v) Tween-20 (Sigma, France). Nonspecific binding sites were blocked with BSA 1.5% in PBS, for 2h at room temperature. After three 5 min-wash with PBS/Tween 0.05%, 100 µL of serum sample (diluted 1 to 100 in PBS/Tween 0.05%, BSA 1.5%) were added in each well and incubated over night at room temperature or 2 h at 37°C. A 'no primary antibody' served as negative control where sera were replaced by equal volume of PBS. Unbound antibodies were removed off by washing wells 4 times with PBS containing 0.05% (v/v) Tween-20. The bound IgG were detected by a further 60-min incubation with Ig goat anti human IgG /biotin conjugate (Southernbiotech) (1:15000) with subsequent quantification of streptavidin-alcaline phosphatase (1:500 in PBS/Tween 0.05%) using para-nitrophenyl phosphate. Colour development was measured spectrophotometrically at 405 nm on a titreteck multiscan plate reader (model 680 BIORAD) after 30 min incubation in the dark and addition of NaOH 3N. All assays were run in duplicate. Each plate always had a blank value obtained from the optical density given by the diluting medium, a standard highly positive control sample from a SLE patient and a negative control comprised of 50 healthy normal volunteers.The mean + 3 SD of 50 control sera was taken as the threshold for positivity. For each group, the percentage of patients with anti-VE cadherin IgG titer that exceeds the mean control value by more than 3 SDs is shown.

***Neutralisation assay of ELISA:*** Dilutions of SLE serum were preincubated overnight at 4°C with recombinant protein EC 1-4 in concentrations varying from 0·5 to 10 µg/ml. The next morning, the samples were transferred to polystyrene microtitre plates coated with EC1-4 and the ELISA was performed as above. Results were considered positive when 50% inhibition was achieved.

***Statistical Analysis:*** All results are expressed as the mean ± SEM. All experiments were performed at least in triplicate to ensure that similar results were obtained in multiple experiments. Differences between the data sets were evaluated by the Mann-Whitney U -test. For all analysis, p<0.05 confidence interval (95% CI) was calculated for percentage data.

### Results

***Production of recombinant VE***-***cadherin fragments for the detection of VE-cadherin autoantibodies:*** The most obvious assays for antibodies detection is a specific ELISA. The development of an ELISA requires the availability of pure antigen preparation. The purification of large amounts of pure VE-cadherin from natural sources is technically difficult, particularly with respect to reproducibility of the preparation. Therefore, we decided to produce a recombinant form in bacteria. The fragments spanning different extracellular repeats of the protein VE-cad chimeras EC1 (1-151 AA), EC1-2 (1-258 AA), EC1-3 (1-372 AA), EC 3-4 (212-431 AA), EC1-4 (1-431 AA) were produced and purified in the laboratory according to the protocol described in (21). Their sequence is illustrated in Figure 1. In a first step the relevant epitopes on the protein had to be characterized. SDS-PAGE analysis of the samples was performed to assess their purity as well as their molecular weight which were compared with the theoretical weights. The samples were stored in a 8M urea to ensure their stability. Validation of the recombinant fragments immunoreactivity by western blot was performed using a rabbit polyclonal anti EC1 antibody.

***ELISA assay for Detection of VE-cadherin autoantibodies:*** We first developed the ELISA with the recombinant fragment EC 1-4 of the extracellular domain of human VE-cadherin. This fragment was coated to microtiter plates as described in *Materials and Methods.* The immunoreactivity was first tested with increasing dilutions of a VE-cadherin monoclonal antibody (BV9) which gives a reasonable slope of the calibration curve (Figure 2A). With then determined the serum dilution to be used for the screening of all the population of SLE patients. A first assay was performed using serum dilutions of two SLE patients and one healthy donor starting from 1 to 50 till 1 to 3200. The binding of autoantibodies to the EC1-4 fragment was detected by a peroxidase-conjugated anti-human IgG antibody. The results show the detection of AAVE in dilution 1:50 in 1:800 for both serums of SLE patients, while the other dilutions were negative. Using the linear regression line, the dilution 1:200 was determined to be the more accurate (Figure 2C), (r=0.997 for SLE#1; 0.9867 for SLE#2 and 0. 9772 for the healthy donor). The dilution 1:200 was then used for all the assays in this study. Antibodies to-VE-cadherin in two SLE patients were characterized for their specificity for the antigen EC1-4. The pre-incubation of the serum with increasing concentrations of antigen CAD1-4 before ELISA, showed a decreased immunocapture which is in favour of a specificity of the human IgG for the fragment CAD1-4 (Figure 2D).

***Purification of the IgG of patient SLE's serum:*** Patient SLE's serum exhibiting a high concentration of VE-cadherin antibodies anti-VE and a sufficient volume (500µl) was used to isolate total IgG fractions by ProteinG-sepharose affinity chromatography as described in Material and Methods. The eluted proteins were analyzed by SDS-PAGE. Eluted proteins by glycin exhibited a molecular weight 50kDa and 20kDa corresponding to the heavy and light chains of the total immunoglobulins (1.25 mg/ml). The same experiment was performed with a healthy donor serum.

***Characterization of the epitopes of two lupic patients' sera and one healthy donor:*** To identify the epitope of the SLE Ig patient, were tested for their capacity to bind the SLE Ig patient Ig by ELISA assay using VE-cad chimeras EC1 (1-151 AA), EC1-2 (1-258 AA), EC1-3 (1-372 AA), EC1-4 (1-477 AA). The recombinants fragments were coated at a stoechiometric concentration (0.2µM) and the ELISA was performed as described in Methods. Increasing dilutions (100, 200, 400 and 800) of purified IgG from SLE and healthy subject were tested in ELISA. Findings from ELISA analysis have been represented in Figure 3 depict the immunoreactivity of patient's sera testing positive in ELISA with EC1-4, EC3-4, and to a lesser extent EC1 and EC1-2. No immunoreactivity was found with healthy donor. Dot blot analysis was then performed with recombinants fragments as described in Methods, and incubated with SLE purified IgG. A positive control of the experience was performed by incubation of an identical membrane with the monoclonal antibody BV9 directed to human VE-cadherin. The SLE purified IgG (240µg / mL) allowed the detection of all the VE-cadherin recombinants. No signal was detected with purified IgG from the healthy donor suggesting that the dot blot analysis might be suitable for the demonstration of the presence or absence of autoantibodies.

*Endothelial cell-cell dissociation upon **SLE** purified Ig **challenge:*** We next investigated whether these autoantibodies to VE-cadherin had an effect on endothelium using classical tests in vitro with endothelial cells in primary culture (HUVEC). The assay is classical for VE-cadherin antibodies and has been reported in (21). Briefly, cells are seeded on fibronectin-coated glass coverslips and grown to confluence. Ig from the SLE patient with the highest titer of VE-cadherin autoantibodies were purified on G-Sepharose column and applied to HUVEC monolayer overnight. IgG from one healthy donor were used as control. When added in vitro to an established confluent endothelial cell monolayer, IgG from healthy donor did not affect endothelial cell-to-cell contacts, while purified IgG from SLE patient disturb the confluent monolayer. This result strongly suggests that these autoantibodies to VE-cadherin might contribute to vascular injury in several diseases.

***Prevalence of VE cadherin-specific antibodies in dysimmune disease: study in RA***, ***SLE and sclerodermia patients populations:*** Determination of the positivity cut-off. 50 healthy donors were analyzed to determine the positive cut-off. The mean age of this group was 26 years with a range from 18 to 55 years (Table 1).
As expected, the distribution of the antibody titers was highly skewed to the left. The mean OD (±SD) values obtained for anti-IgG in 1:200 diluted serum from 50 healthy donors were 0.38 ± 0.17. The OD positivity cut-offs were determined with 1:200 diluted serum as the mean + 3 SD; this value was 0.77. The inter-assay coefficient of variation (CV) for ten runs with the positive control was 12.5%. Samples were considered positive when the OD value was higher than cut-off (>0.77 for anti-IgG).

***Population analysis:*** We then analyzed a RA, SLE and sclerodermia patients populations. The patient population is illustrated in Table 1. The rate of VE-cadherin autoantibodies was significantly higher in 63RA patient's sera from Rouen hospital (Pr Olivier Vittecocq PHRC VERA). 95,8% of RA patients were positive for VE-cadherin autoantibodies (p< 0.005). In SLE group (n=35), the threshold allowed to discriminate 49% of the positive patients in VE-cadherin auto antibodies (Mann-Whitney p < 0,001). The patient serums of scleroderma (Scl) population exhibited 19% of positive patients for VE-cadherin autoantibodies (Mann-Whitney p<0.001) (Figures 4A, B).

***Evidence for VE-cadherin autoantibodies in patient's sera positive for anti-endothelial cell antibodies AECA:*** AECA have been detected in most of autoimmune and inflammatory diseases in autoimmune and systemic inflammatory diseases, including systemic vasculitis. Indeed, AECA that bind to human endothelial cells cultured in vitro have been detected in a variety of autoimmune diseases with vascular pathology. An indirect inmmunofluorescence assay using cultured HUVECs represented one of the major methods for AECA detection but lacked reproductibility and specificity (27). We thus examined wether these patient sera were positive for VE-cadherin auto antibodies. The ELISA was performed using EC 1-4 as antigen for two sera positive for AECA. We found that both sera were positive for AAVE as compared to negative healthy donor (Figure 5). Using the different VE-cadherin chimeras, it was found that the two AECA positive patient preferentially detected EC1-4>EC 1-3. It is noteworthy these epitopes looked slightly different from those observed in SLE patients. These results suggest that VE-cadherin chimera ELISA might be of interest for future analysis for which AECA diagnosis were not reproducible or sensitive. Prospective analysis is needed to confirm this hypothesis.

### EXAMPLE 2: CHARACTERISATION OF ANTI-VE-CADHERIN ANTIBODIES IN A PATIENT WITH NEUROLOGICAL BEHÇET'S DISEASE

Behçet's disease is a vasculitis affecting all sizes of arteries and veins. A change in endothelial cell phenotype plays a key role in its pathogenesis and contributes to the rise in leukocyte diapedesis, adverse changes in NO-dependent arterial vasodilatation and to hypercoagulability.

### Material & Methods

***Subjects studied:*** We describe the case of a female patient suffering from Behçet's disease from our series of patients with Behçet's disease and ocular involvement. We have undertaken a biological characterisation of anti-VE-cadherin antibodies in this patient. We chose this patient as she had two specific features: central neurological involvement and an anti-VE-cadherin antibody titre 6.5 times greater than the mean in healthy people.

***ELISA measurement of anti-VE-cadherin antibodies:*** Five micrograms of the recombinant VE-EC1-4 fragment were coated in 96 ELISA plate wells and incubated overnight at 4°C. The wells were washed and saturated for 2 h at 37°C with 1.5% BSA. Two hundred microlitres of patient plasma diluted 1/100 in PBS-Tween-BSA were added for 2 hours at 37°C. The wells were then washed and 100 µl of a biotinylated goat anti-human IgG Ig solution (Southernbiotech) were added at a concentration of 1/5000 and incubated for 1 hour at room temperature. Streptavidin bound to alkaline phosphatase was added and the enzyme activity was then measured by adding the substrate pNNP. Optical densities were read at 10 min at 405 nm.

***Affinity chromatography purification of IgG:*** Serum from the Behçet's patient (4 ml) was diluted in 36 ml of NaH2PO4, Na₂HPO₄ 0.2M, pH 7.1 phosphate buffer and was incubated with G protein-Sepharose (2 ml) (previously equilibrated with 12 ml of NaH2PO4, Na₂HPO₄ 0.2M, pH 7.1 phosphate buffer) in a Falcon tube overnight at 4°C, gently shaking. At the end of the incubation the gel was poured into a Biorad column (1 x 10 cm) and the filtration fraction was retained. After rinsing the column with 60 ml of the same buffer, 10 ml of 100 mM glycine buffer pH 8 were added to elute the column. 1 ml fractions were collected into Eppendorf tubes containing 100 µl of Tris 1mM, pH 8. Optical densities were read at 280 nm in an aliquot of each 1 ml fraction collected to establish the IgG concentration (OD = ε x C x L (OD = optical density; ε = molecular absorption coefficient (εIg = 1.45 1/g/cm); C = concentration of the solution (g/l); L = length of cuvette (cm)). The final fractions were dialysed against PBS overnight at 4°C before use. The same protocol was followed to purify the IgG from a healthy donor.

***Western-Blot immunodetection of recombinant fragments EC1-4, EC1, EC1-2, EC1***-***3 and EC3-4:*** The recombinant fragment was separated by 12% or 15% (SDS-PAGE), polyacrylamide gel electrophoresis and then transferred to a nitrocellulose membrane (110V, 1h). After saturating the membrane with PBS-Tween-milk it was incubated with the purified patient IgG fractions (250 µg/ml or 50 µg/ml) in a saturating medium (PBS-tween-milk). Bound IgG was revealed by adding human anti-IgG antibodies (A2290 sigma) bound to peroxidase and using the ECL kit (electrochemoluminescence).

***Specificity of anti***-***VE***-***cadherin antibody assay:* A**. Dilution: dilutions of the Behçet-IgG and healthy donor IgG purifications were produced. Two hundred microlitres of the purified serum diluted at 1/100, 1/200, 1/400, 1/800 in PBS-Tween 0.05% were placed in the wells. **B.** Competition with the recombinant EC1-4 fragment: 1/100 and 1/400. Increasing concentrations of EC1-4 (17.8 µg/ml, 8.89 µg/ml, 4.495 µg/ml, 2.25 µg/ml, 1.125 µg/ml) were added to the Behçet's patient IgG diluted 1/100 or 1/400 in PBS-Tween-BSA 1.5%.

***Cell culture:*** HUVEC cells were isolated using the method previously described. Only HUVEC cells from a first to a third passage were used.

***Immunofluorescence:*** In order to examine the cells by immunofluorescence microscopy, they were cultured on glass coverslips. The coverslips (diameter 13 ram.) were coated overnight at 4°C, with 7 mg/ml of fibronectin and then washed with a serum-free culture Medium 199. 3.5 x 10⁴ cells/slide were placed in 0.5 ml of culture medium. The HUVEC were cultured to confluence for 72 h. Cell monolayers were incubated overnight at 37°C, with IgG purified from a Behçet patient serum (2.5 mg/ml) and from a healthy donor. The cells were fixed with 3.5% (p/v) paraformaldehyde, PBS, for 20 minutes at room temperature and then washed 3 times with PBS. They were rendered permeable with methanol at -20°C for 10 minutes followed by incubating for 10 minutes in Triton X-100 (0.5% in PBS). The cells were rinsed 3 times with PBS and non-specific binding sites were saturated with PBS/1 mg/ml of BSA for 30 minutes. They were incubated with primary antibody (polyclonal rabbit anti-VE-cadherin antibody described previously (14)) in PBS at room temperature for 1 hour. After washing 3 times with PBS+, the cells were incubated for 1 h with a cyanine 3 rabbit anti-IgG conjugated antibody (1:1000). They were washed again 3 times with PBS and the nuclei were then labelled with 1 µg/ml of Hoechst for 5 minutes and then washed 3 times with PBS. The cover slips were rinsed and then dried with ethanol and mounted onto Mowiol 4-88 slides (Hoechst, Frankfurt, Germany). The cells were examined through a Zeiss Axioskop 2 microscope (equipped with an AxioCam CCD colour camera (Zeiss)). Images were digitalised by AxioVision software (Zeiss).

### Results

***Characterisation of the anti- VE-cadherin antibodies by Western-Blot and ELISA:*** As described previously in our series of Behçet's patients with ocular involvement, the ELISA revealed anti-VE-cadherin (EC1-4) antibodies in patients suffering from Behçet's disease at significantly higher titres (Mann-Whitney U test, p = 0.00021) than in the healthy population. The patient we described above had a very high anti-VE-cadherin (EC1-4) titre of 1.47. Her total IgG was purified by affinity chromatography on a G-Sepharose column. We carried out the same procedures on serum from a healthy subject with a low anti-VE-cadherin (EC1-4) antibody titre of 0.29.
Anti-VE-cadherin (EC1-4) IgG were demonstrated by Western-Blot in the IgG purified from our patient's serum and were not found in the healthy subject after purification using the same method. Increasing dilutions (100, 200, 400, 800) of purified IgG from our Behçet's patient and from the healthy subject were tested by ELISA. We found dose-dependent binding of the IgG to the EC1-4 antigen coated onto the plate. Pre-incubating the purified IgG with increasing concentrations of EC1-4 antigen before the ELISA reduced the immunocapture, suggesting that the human IgG were specific for the EC1-4 fragment.

***Molecular identification of the idiotypes of the anti-VE-cadherin antibodies isolated:*** In order to establish the idiotypes of the anti-VE-cadherin antibodies we ran different recombinant fragments of the extracellular domain of VE-cadherin on SDS-PAGE. Immunodetection was performed by Western-Blot with purified IgG from the Behçet's patient (50 µg/ml). The patient's purified IgG antibodies recognised the recombinant fragments EC1-4 and EC3-4 and not fragments EC1, EC1-2, EC1-3. We then carried out an ELISA with 10 µg of each recombinant fragment coated onto different wells. The wells were incubated with increasing dilutions of the Behçet plasma. The optical densities obtained with EC1-4 and EC3-4 were greater (4.7 times) than those obtained with EC1, EC1-2, EC1-3. The idiotypes of the anti-VE-cadherin antibodies are directed against the extracellular 3-4 domain.

***Effect of a purified IgG from a Behçet's patient on a HUVEC cell culture:*** The HUVEC culture incubated with IgG purified from a healthy subject displayed homogeneous labelling of VE-cadherin with a polyclonal rabbit anti-VE-cadherin antibody showing the adherens junctions. The HUVEC culture incubated with IgG purified for the Behçet's patient exhibited reduced immunolabelling of the VE-cadherin by the polyclonal rabbit anti-VE-cadherin antibodies and the distribution of labelling became heterogeneous.

We have thus demonstrated for the first time the presence of anti-VE-cadherin antibodies targeting the EC3-4 part of the extracellular domain of the protein in a patient suffering from spinal neurological Behçet's disease. We have shown that purified IgG from the patient reduced and disorganised VE-cadherin labelling in a HUVEC culture.

### EXAMPLE 3: DETECTION OF ANTI-VE-CADHERIN AUTOANTIBODIES IN SERA OF PATIENTS AFFECTED WITH CANCER DISEASES

### Material & Methods

***Sources of human sera:*** A retrospective study was performed on patients enrolled from 1999-2008 including:
- 59 Breast cancer patients (29 patients with metastatic breast cancer and 30 patients with localized breast cancer). All patients were treated in a single institution between 1999 and 2007 (Leon Berard Center, Lyon, France); and
- 10 Metastatic Kidney cancers patients were enrolled between 1999 and 2002 in Leon Berard Center, Lyon FRANCE, and treated in this institution (PERCY Protocol).

**Table 2: Characterization of patient population**

| **Disease** | **No**. | **Median age (range)** | **Female**/**male** |
|---|---|---|---|
| Breast cancer | 60 | 55.4 (35-75) | 60/0 |
| Kidney cancer | 10 | 59.1 (44-77) | 2/10 |

It should be further noted that said sera obtained from cancer patients were analyzed as previously described in Examples 1 and 2.

### Results

Using an ELISA with recombinant VE-cad(1-4) fragments encompassing four extracellular NH2-terminal domains of VE-cadherin, we detected anti VE-cadherin auto antibodies in sera from renal cancer patients (92%) and breast cancer patients (70%), , while no antibodies were found healthy patients (0%).

### EXAMPLE 4: DETECTION OF ANTI-VE-CADHERIN AUTOANTIBODIES IN CEREBROSPINAL FLUID (CSF) OF PATIENTS AFFECTED WITH NEUROLOGICAL BEHÇET'S DISEASE

Neurobehçet disease (NBD) is a rare complication of Behçet disease (BD) but with important burdens of morbidity and mortality. Little is known about this complication because there are no validated diagnostic criteria, and all the studies have small number of patients. The prevalence reported normally ranges between 5% and 15% and it is more frequent amongst men between 20 and 40 years old.

### Material & Methods

***Patients with Behçet's disease:*** As part of the Behçet's disease laboratory markers study (AFSSAPS number: 2007-A00430-53) sponsored by the Grenoble University Hospital, all patients with Behçet's disease with ocular involvement were included between January 2008 and February 2009. All included patients met diagnostic criteria for Behçet's disease (ICBD) proposed in 2006. General clinical data were standardised using a completed form at baseline inclusion including age, sex, clinical features, HLA-B51 status.

***Case report:*** A 50 years old Algerian female patient who suffered from anterior uveitis of the right eye treated with local corticosteroid therapy which relapsed as posterior uveitis. She had recurrent buccal aphthous ulceration and pseudofolliculitis skin lesions. The patient developed right knee arthritis associated with polyarthralgia (sacro-iliac, wrists and hands). According to the diagnosis of Behcet disease without initial neurological involvement, she was treated in 2004 with systemic corticosteroids combined with colchicine and methotrexate. The patient decided to stop taking treatments on her own initiative because of their sides effects. Two years later, the patient developed neurological syndrom associated with difficulty walking, altered sensation, thigh and calves pains with electrical discharge. Clinical examination showed central disorders: lower limb hyperreflexia, reduced proprioceptive and extra-lemniscal sensation. The disease was progressive over 18 months and the patient cannot walk. A cerebro-spinal magnetic resonance imaging showed increased T2 signals in the supratentorial white matter associated with increased signals in the spinal cord in a T2 sequence, intramedullary at C2 and anterior at C3-C4-C5 supporting the diagnosis of neuro Behçet's disease

***ELISA for detection of VE cadherin auto antibodies in CSF of three patients:*** Different CSF concentrations were analyzed by ELISA (1/10 to 1/160). For each patient, the average of two OD is represented by a single point for each dilution. CSF from patients presenting a Primary Central Nervous System Vasculitis (PCMSV), a neuroBehcet disease were tested, comparating with an healthy CSF.

### Results

***CSF analysis:*** We have previously identified the presence of VE-cadherin autoantibodies (AAVE) in a Behcet patient serum. This patient exhibited a neurological syndrome. We thus further investigated the biology of cerebrospinal fluid. We found the presence of AAVE in the CSF of this patient while no AAVE were detected in a healthy patient. Another patient exhibiting a Primary Central Nervous System Vasculitis was found to have a high concentration of AAVE in its CSF. This is the first description of AAVE in CSF from patients exhibiting neurological syndroms.

### EXAMPLE 5: DETECTION OF ANTI-VE-CADHERIN AUTOANTIBODIES IN SERA OF PATIENTS AFFECTED WITH BIRDSHOT DISEASE

Birdshot chorioretinopathy (BSCR) is a bilateral intraocular inflammatory disease characterized by vitritis and multiple cream-colored fundus lesions.

### Material & Methods

***Patients with Birdshot disease:*** A cohort of patients the disease followed Birdshot at Grenoble University Hospital, Department of Ophthalmology (n = 28, 13 men, 15 women) was analyzed. Patients receive an annual consultation, in which a comprehensive paraclinical is performed and a blood sample is collected. The sera obtained were compared to sera of healthy patients (n = 92) from the EFS.

***Fluorescein angiography:*** Intravenous injection (in antecubital vein) of fluorescein (AK-Fluor 10%) to assess the state of retinal vascularisation. Follow by dilatation of pupils, to show the retinal vascularisation. Series of photographs of the fundus are taken using a blue filter, between 0 and 15 min after injection. This allows to see its passage through the arterial and venous retinal vessels, and realizes a dynamic study of the retinal vascularisation.

### Results

***Analysis of Birdshot patients:*** The anti-VE-cadherin autoantibodies were detected in Birdshot disease (m=0.5035) at a rate higher than the cohort of healthy donors (m=0.4396), but not statistically significant (p = 0.39). However, it was observed a particularly high level of anti-VE-cadherin autoantibodies in 3 patients with advanced, characterized in angiography extravasation of contrast material, indicating a disruption of the endothelial barrier. Flow and permeability in the retinal and choroidal vessels are correlated with anatomical changes, showed by fluorescein angiography. High rate of anti-VE-cadherin autoantibodies are correlated with high lesions in angiography.

The quantification of anti VE cadherin antibodies could be a biomarker of the severity of the vasculitis in Birdshot chorioretinopathy. It could be a non-invasive marker of vasculitic lesion for patient who can't have angiography (allergy, refusal), without any side effect (nausea, yellow skin or secretion). If this correlation is extended to any vasculitis, it could be a biomarker of severity of the disease, and maybe a prognosis factor, which lead to a more invasive therapy.The angiographic slides showed Multiple leak of fluorescein in retinal arteries, suggesting of severe retinal vasculitis. This correlated with an high level of anti VE cadherin auto antibodies (OD = 0.9355) Normal retinal vasculature, correlated with a low level of anti VE cadherin auto antibodies (OD = 0.3035).

### DISCUSSION:

The goal of our research was to consider that the major protein from endothelial adherens junctions (VE-cadherin) exhibit structural modifications and thus leaky junctions in several inflammatory and angiogenic processes which is not the case in adult quiescent endothelium (16, 17), and to hypothesize that autoantibodies might exist in autoimmune and inflammatory diseases. In addition, our work was based on the assumption that antibodies directed against certain epitopes may have a higher diagnostic potential. Thus, the overall aim of our study was to provide a framework for the future development of a new assay for the detection of VE-cadherin autoantibodies.

An ELISA, based on the human recombinant VE-EC1-4 fragment, was first developed and tested with a VE-cadherin antibody (BV9) and with SLE patients, as this autoimmune disease was characterized for its high level of several autoantibodies. The ELISA yields a numerical result, which permits an unbiased interpretation. The technique allowed detection on serum diluted 1:200 and was specific for EC1-4.

Immunopurified IgG from healthy donor and SLE patients were tested by ELISA versus several VE-cadherin fragments. These tools could allow to determine potential specific epitopes for dysimmune diseases. For two SLE patients, it seems that EC1 might be the target of these IgG. This experiment was confirmed by dotblot which is a faster technique that can be further used in biological clinical practises.

We reasoned that in human dysimmune diseases, the presence of VE-cadherin autoantibodies could directly target the patient blood vessel. Our preliminary data support this hypothesis as we found that SLE purified IgG were able to dissociate endothelial cell monolayer. This experiment required a higher volume of blood than ELISA, thus it could not be performed for the 35 SLE patients. It will be of importance to draw prospective clinical studies to determine the function of these VE-cadherin autoantibodies and their link with the physiopathology.

Our study of patient population provides evidences for a strong prevalence of VE-cadherin autoantibodies in RA, SLE and to a lesser extent in SCL patients. The analysis of a large number of patients is further needed in prospective studies to validate the evidence for VE-cadherin autoantibodies in dysimmune diseases before definitive conclusions about the disease association of VE-cadherin autoantibodies can be drawn. Of importance, it will be of interest to examine the potential association between anticardiolipin antibodies and lupus anticoagulants with venous, and possibly, arterial thrombosis and VE-cadherin autoantibodies.

Finally, the presence of VE-cadherin autoantibodies were detected in sera from patients that were positive in AECA. The concept of antibodies with endothelial cell specificity was introduced in the context of autoimmune diseases, including scleroderma, antiphospholipid antibody, Behçet disease, lupus erythematosus, and Schönlein-Henoch purpura (31-33). They have been detected in up to 74% of patients with SLE (29). It always has been questioned whether anti-endothelial cell antibodies are of any pathogenetic significance. Previous studies have shown that IgG or IgM from patients with results positive for anti-endothelial cell antibodies were capable of causing antibody-dependent cellular cytotoxicity of HUVECs (34). Wether or not VE-cadherin might represent a new specific antigen for AACE needs further investigations.

Altogether, it is noteworthy that autoantibodies to human VE-cadherin in patients' serum have never been studied and described. Thus our study will bring a method and the tools to detect VE-cadherin autoantibodies in patient serum and to determine the prevalence and clinical significance of these autoantibodies in dysimmune diseases and cancer. In addition, it will be important to determine the origin of anti-VE-cadherin antibodies. The question to be addressed of whether there are other relevant epitopes within the different VE-cadherin chimeras. Clearly, we are still at the beginning of further investigations of this interesting phenomenon. Particularly, it is still unclear whether these antibodies are the cause of disease processes or only markers for a disease.

In conclusion, due to the seriousness of autoimmune and inflammatory disorders and the risk to develop severe vascular complications, an early diagnosis would be, obviously, extremely desirable. Unfortunately, no method for diagnosing or monitoring such disorders as well as for determining the risk to develop vascular complications exists that is entirely satisfactory and completely informative. Thus, the ELISA for the detection and quantification of anti-VE-cadherin autoantibodies, described here, is probably a tool for the systematic examination of clinical correlations between VE-cadherin antibodies and disease occurrence and /or progression. It is expected to demonstrate the clinical relevance of these autoantibodies as potential biomarkers for diagnosis and predicting the vascular alterations associated with these dysimmune diseases. It may also be useful in therapeutic decision-making, and in following the time course of autoantibodies during treatment.

### REFERENCES:

Throughout this application, various references describe the state of the art to which this invention pertains.
1. Davidson A, Diamond B. Autoimmune diseases. N Engl J Med. 2001 Aug 2;345(5):340-50.
2. Weyand CM, Goronzy JJ. Medium- and large-vessel vasculitis. N Engl J Med. 2003 Jul 10;349(2):160-9. Review.
3. Charo IF, Ransohoff RM. The many roles of chemokines and chemokine receptors in inflammation. N. Engl J Med. 2006 Feb 9;354(6):610-21. Review.
4. Gibbons GH, Dzau VJ The emerging concept of vascular remodeling, N Engl J Med, 1994, 330, 20, 1431-1438
5. Yap AS, Brieher WM, Gumbiner BM. Molecular and functional analysis of cadherin-based adherens junctions.Annu Rev Cell Dev Biol. 1997;13:119-46.
6. Lampugnani M. G., Resnati M., Raiteri M., Pigott R., Pisacane A., Houen G., Ruco L. P., Dejana E. A novel endothelial-specific membrane protein is a marker of cell-cell contacts (1992) J. Cell Biol. 118:1511-1522.
7. Lampugnani MG. The molecular organization of endothelial cell to cell junctions, differential association of plakoglobin, -catenin, and -catenin with vascular endothelial cadherin (VE-cadherin). J Cell Biol. 1995;129: 203-217
8. Vestweber D. VE-CADHERIN. The major endothelial adhesion molecule controlling cellular junctions and blodd vessel formation. Arterioscler Thromb Vasc Biol 2008; 28: 223-32
9. Breviario F., Caveda L., Corada M., Martin-Padura L., Navarro P., Golay J., Introna M., Gulino D., Lampugnani M-G., Dejana E. Functional properties of human vascular endothelial cadherin (7B4/cadherin-5), an endothelium-specific cadherin. Artherioscler. Throm. Vasc. Biol., 15: 1229-1239, 1992.
10. Carmeliet P., Lampugnani M. G., Moons L., Breviario F., Compernolle V., Bono F., Balconi G., Spagnuolo R., Oosthuyse B., Dewerchin M., Zanetti A., Angellilo A., Mattot V., Nuyens D., Lutgens E., Clotman F., Ruiter M. A. D., Groot A. G., Poelmann R., Lupu F., Herbert J. M., Collen D., Dejana E. Targeted deficiency or cytosolic truncation of the VE-cadherin gene in mice impairs VEGF-mediated endothelial survival and angiogenesis. Cell, 98: 147-157, 1999.
11. Caveda L., Martin-Padura I., Navarro P., Breviario F., Corada M., Gulino D., Lampugnani M. G., Dejana E. Inhibition of cultured cell growth by vascular endothelial cadherin (cadherin-5/VE-cadherin). J. Clin. Investig., 98: 886-893, 1996
12. Corada M., Mariotti M., Thurston G., Smith K., Kunkel R., Brockhaus M., Lampugnani M. G., Martin-Padura I., Stoppacciaro A., Ruco L., Dejana E. Vascular endothelial-cadherin is an important determinant of microvascular integrity in vivo. Proc. Natl. Acad. Sci. USA, 96: 9815-9820, 1999.
13. Bach T. L., Barsigian C., Chalupowicz D. G., Busler D., Yaen C. H., Grant D. S., Martiney J. VE-Cadherin mediates endothelial cell capillary tube formation in fibrin and collagen gels. Exp. Cell Res., 238: 324-334, 1998.
14. Gory-Fauré S, Prandini MH, Pointu H, Roullot V, Pignot-Paintrand I, Vernet M, Huber P. Role of vascular endothelial-cadherin in vascular morphogenesis. Development. 1999;126(10):2093-102.
15. Vittet D, Buchou T, Schweitzer A, Dejana E, Huber P. Targeted null-mutation in the vascular endothelial-cadherin gene impairs the organization of vascular-like structures in embryoid bodies. Proc Natl Acad Sci USA. 1997;94(12):6273-8.
16. Lambeng N, Wallez Y, Rampon C, Cand F, Christe G, Gulino-Debrac D, Vilgrain I, Huber P. Vascular endothelial-cadherin tyrosine phosphorylation in angiogenic and quiescent adult tissues. Circ Res 2005, 96: 384-91
17. Hudry-Clergeon H, Stangel D, Ninio E, Vilgrain I. Platelet-activating factor increases VE-cadherin tyrosine phosphorylation in mouse endothelial cells and its association with the PtdIns3'-kinase. FASEB J 2005; (6): 512-20.
18. Wallez Y, Cand F, Cruzalegui F, Wernstedt C, Souchelnytskyi S, Vilgrain I, Huber P. Src kinase phosphorylates vascular endothelial-cadherin in response to vascular endothelial growth factor: identification of tyrosine 685 as the unique target site. Oncogene. 2007;26(7):1067-77.
19. Hermant B, Bibert S, Concord E, Dublet B, Weidenhaupt M, Vernet T, Gulino-Debrac D. Identification of proteases involved in the proteolysis of vascular endothelium cadherin during neutrophil transmigration.J Biol Chem. 2003 Apr 18;278(16):14002-12.
20. L Bouillet, T Mannic, M Arboleas, M Subileau, C Massot, C Drouet, P Huber, I Vilgrain Hereditary angioedema: Key role for kallikrein and bradykinin in vascular endothelial-cadherin cleavage and edema formation. JACI 2011, online 25 March 2011.
21. Gulino D, Delachanal E, Concord E, Genoux Y, Morand B, Valiron MO, Sulpice E, Scaife R, Alemany M, Vernet T. Alteration of endothelial cell monolayer integrity triggers resynthesis of vascular endothelium cadherin. J Biol Chem. 1998 Nov 6;273(45):29786-93.
22. Corada M, Zanetta L, Orsenigo F, Breviario F, Lampugnani MG, Bernasconi S, Liao F, Hicklin DJ, Bohlen P, Dejana E. A monoclonal antibody to vascular endothelial-cadherin inhibits tumor angiogenesis without side effects on endothelial permeability. Blood. 2002;100(3):905-11.
23. Liao F, Doody JF, Overholser J, Finnerty B, Bassi R, Wu Y, Dejana E, Kussie P, Bohlen P, Hicklin DJ. Selective targeting of angiogenic tumor vasculature by vascular endothelial-cadherin antibody inhibits tumor growth without affecting vascular permeability. Cancer Res. 2002;62(9):2567-75.
24. Corada M, Liao F, Lindgren M, Lampugnani MG, Breviario F, Frank R, Muller WA, Hicklin DJ, Bohlen P, Dejana E. Monoclonal antibodies directed to different regions of vascular endothelial cadherin extracellular domain affect adhesion and clustering of the protein and modulate endothelial permeability. Blood. 2001;97(6):1679-84.
25. Liao F, Li Y, O'Connor W, Zanetta L, Bassi R, Santiago A, Overholser J, Hooper A, Mignatti P, Dejana E, Hicklin DJ, Bohlen P. Monoclonal antibody to vascular endothelial-cadherin is a potent inhibitor of angiogenesis, tumor growth, and metastasis. Cancer Res. 2000;60(24):6805-10.
26. Kallenberg CG, Heeringa P, Stegeman CA. Mechanisms of Disease: pathogenesis and treatment of ANCA-associated vasculitides. Nat Clin Pract Rheum. 2006 ; 2(12):661-70.
27. Servettaz A, Guilpain P, Tamas N, Kaveri SV, Camoin L, Mouthon L. Natural anti-endothelial cell antibodies. Autoimmun Rev. 2008 Jun; 7(6):426-30.
28. Cines DB, Lyss AP, Reeber M, et al. Presence of complement-fixing anti-endothelial cell antibodies in systemic lupus erythematosus. J Clin Invest. 1984;73:611-625.
29. Shingu M, Hurd ER. Sera from patients with systemic lupus erythematosus reactive with human endothelial cells. J. Rheumatol. 1981;8:581-586.
30. Garnier-Raveaud S, Usson Y, Cand F, Robert-Nicoud M, Verdetti J, Faury G. Identification of membrane calcium channels essential for cytoplasmic and nuclear calcium elevations induced by vascular endothelial growth factor in human endothelial cells. Growth Factors. 2001; 19: 35-48.
31. Belizna C, Duijvestijn A, Hamidou M, Tervaert JW. Antiendothelial cell antibodies in vasculitis and connective tissue disease. Ann Rheum Dis. 2006 ;65(12):1545-50.
32. Mihai C, Tervaert JW. Anti-endothelial cell antibodies in systemic sclerosis. Ann Rheum Dis. 2010;69(2):319-24.
33. Youinou P, Le Dantec C, Bendaoud B, Renaudineau Y, Pers JO, Jamin C. Endothelium, a target for immune-mediated assault in connective tissue disease. Autoimmun Rev. 2006 Mar;5(3):222-8.
34. Cines DB, Lyss AP, Reeber M, et al. Presence of complement-fixing anti-endothelial cell antibodies in systemic lupus erythematosus. J Clin Invest. 1984;73:611-625.).

## Claims

1. A method for predicting and/or diagnosing vascular alterations associated with a disorder in a patient, comprising a step of detecting or quantifying the presence of anti-VE-cadherin autoantibodies in a biological sample obtained from said patient.

2. The method according to claim 1, wherein the step of detecting or quantifying the presence of anti-VE-cadherin autoantibodies is carried out by using at least one VE-cadherin antigen.

3. The method according to claim 2, wherein the VE-cadherin antigen is the VE-cadherin extracellular domain or a fragment thereof.

4. The method according to anyone claims 2 to 3, wherein the VE-cadherin antigen is immobilized onto a solid support.

5. The method according to anyone of claims 1 to 4, wherein the step of detecting or quantifying the presence of anti-VE-cadherin autoantibodies is carried out by an enzyme immunoassay or enzyme-linked immunoassay (EIA or ELISA).

6. The method according to anyone of claims 1 to 5, wherein the biological sample is a serum sample.

7. The method according to anyone of claims 1 to 6, wherein the disorder is selected in the group consisting of cancer disorders and dysimmune diseases.

8. A kit suitable for carrying out the method according to anyone of claims 1 to 7, comprising a solid support coated with at least one VE-cadherin antigen and at least a labelled antibody specifically recognizing antibodies.

## Patentansprüche

1. Verfahren zum Vorhersagen und/oder Diagnostizieren von vaskulären Veränderungen, die mit einer Störung in einem Patienten assoziiert sind, umfassend einen Schritt des Nachweisens oder Quantifizierens der Anwesenheit von Anti-VE-Cadherin-Autoantikörpern in einer biologischen Probe, welche von dem Patienten erhalten wurde.

2. Verfahren nach Anspruch 1, wobei der Schritt des Nachweisens oder Quantifizierens der Anwesenheit von Anti-VE-Cadherin-Autoantikörpern durch Verwenden von wenigstens einem VE-Cadherin-Antigen durchgeführt wird.

3. Verfahren nach Anspruch 2, wobei das VE-Cadherin-Antigen die extrazelluläre Domäne des VE-Cadherins oder ein Fragment davon ist.

4. Verfahren nach einem der Ansprüche 2 bis 3, wobei das VE-Cadherin-Antigen auf einem festen Träger immobilisiert ist.

5. Verfahren nach einem der Ansprüche 1 bis 4, wobei der Schritt des Nachweisens oder Quantifizierens der Anwesenheit von Anti-VE-Cadherin-Autoantikörpern von einem Enzymimmunassay oder einem enzymgekoppelten Immunassay (EIA oder ELISA) durchgeführt wird.

6. Verfahren nach einem der Ansprüche 1 bis 5, wobei die biologische Probe eine Serumprobe ist.

7. Verfahren nach einem der Ansprüche 1 bis 6, wobei die Störung ausgewählt ist aus der Gruppe bestehend aus Krebserkrankungen und Dysimmunkrankheiten.

8. Kit geeignet zum Durchführen des Verfahrens nach einem der Ansprüche 1 bis 7, umfassend einen festen Träger beschichtet mit wenigstens einem VE-Cadherin-Antigen und wenigstens einem markierten Antikörper, der spezifisch Antikörper erkennt.

## Revendications

1. Procédé de prédiction et/ou de diagnostic d'altérations vasculaires associées à un trouble chez un patient, comprenant une étape de détection ou de quantification de la présence d'auto-anticorps anti-VE-cadhérine dans un échantillon biologique prélevé chez ledit patient.

2. Procédé selon la revendication 1, dans lequel l'étape de détection ou de quantification de la présence d'auto-anticorps anti-VE-cadhérine s'effectue en utilisant au moins un antigène de VE-cadhérine.

3. Procédé selon la revendication 2, dans lequel l'antigène de VE-cadhérine est le domaine extracellulaire de la VE-cadhérine ou un de ses fragments.

4. Procédé selon l'une quelconque des revendications 2 à 3, dans lequel l'antigène de VE-cadhérine est immobilisé sur un support solide.

5. Procédé selon l'une quelconque des revendications 1 à 4, dans lequel l'étape de détection ou de quantification de la présence d'auto-anticorps anti-VE-cadhérine est effectuée par un immunodosage d'enzyme ou un immunodosage lié à une enzyme (EIA ou ELISA).

6. Procédé selon l'une quelconque des revendications 1 à 5, dans lequel l'échantillon biologique est un échantillon de sérum.

7. Procédé selon l'une quelconque des revendications 1 à 6, dans lequel le trouble est choisi dans le groupe constitué des troubles dus au cancer et des maladies dysimmunitaires.

8. Kit convenant pour réaliser le procédé selon l'une quelconque des revendications 1 à 7, comprenant un support solide revêtu d'au moins un antigène de VE-cadhérine et d'au moins un anticorps marqué reconnaissant spécifiquement les anticorps.
